# DEMANDE DE BREVET EUROPEEN

(11) **EP 2 338 908 A1**
(43) Date de publication de la demande: **29.06.2011**
(21) Numéro de dépôt: 10183969.4
(22) Date de dépôt: 18.09.2002
(51) Int. Cl.: C07K 14/47, A61K 38/17, A61P 37/06

(54) **Peptides comportant des modifications de type post-translationnel et leur utilisation dans le cadre du traitement de pathologies auto-immunes**

(30) Priorité: 18.09.2001 FR 0112041
(62) Demande divisionnaire de: 02798771.8
(71) Demandeur: Centre National de la Recherche Scientifique (CNRS), 75794 Paris Cedex 16 (FR)
(72) Inventeur: Muller, Sylviane, 67000 Strasbourg (FR); Monneaux, Fanny, Sylvie, Michèle, 67540 Ostwald (FR); Briand, Jean-Paul, 67000 Strasbourg (FR); Guichard, Gilles, 33170 Gradignan (FR); Guillet, Jean-Gérard, . (FR)
(74) Mandataire: Mouget-Goniot, Claire

(57) **Abrégé**

La présente invention a pour objet de nouveaux peptides transformés de manière à comporter des modifications de type post-traductionnel, telles que phosphorylation ou acétylation d'un ou plusieurs acides aminés. L'invention concerne également leurs procédés d'obtention, et leurs utilisations dans des compositions pharmaceutiques dans le cadre du traitement des pathologies auto-immunes.

## Description

La présente invention a pour objet de nouveaux peptides transformés de manière à comporter des modifications de type post-traductionnel, telles que phosphorylation ou acétylation d'un ou plusieurs acides aminés. L'invention concerne également leurs procédés d'obtention, et leurs utilisations dans des compositions pharmaceutiques dans le cadre du traitement des pathologies auto-immunes.

Plusieurs études ont démontré l'intérêt de l'utilisation de peptides synthétiques pour la prévention dans des modèles murins du développement de pathologies auto-immunes telles que le lupus. Ces études ont été réalisées avec des peptides dérivés soit de séquences d'histones, soit d'anticorps anti-ADN. L'administration par voie intra-veineuse de ces peptides a permis de diminuer la production d'anticorps anti-ADN typiques du lupus et de prolonger la survie des souris traitées. Aucune étude n'a été réalisée à partir de séquences de protéines du splicéosome, autre autoantigène du lupus.

Les quelques études réalisées à ce jour ayant démontré une amélioration de la pathologie lupique chez des souris autoimmunes ont utilisé des peptides ne contenant pas de modifications post-traductionelles (Eilat et al., 2000; Jouanne et al., 1999; Kaliyaperumal et al., 1999; Marino et al, 2000).

Les modifications post-traductionnelles semblent jouer un rôle important dans l'émergence de la réponse auto-immune (Utz and Anderson, 1998). Afin de mettre en place des stratégies d'intervention efficaces, l'identification des cibles réellement responsables de la rupture de tolérance au soi et reconnues ensuite par les cellules autoréactives constitue un intérêt majeur.

L'identification des séquences de la protéine 70K reconnues par les cellules T autoréactives a débuté en 1998. Les inventeurs ont dans un premier temps synthétisé 20 peptides chevauchants couvrant la séquence de la protéine 70K et étudié la reconnaissance de ces peptides par les anticorps de souris lupiques MRL/lpr ainsi que par les cellules T CD4 + autoréactives de ces souris. Parmi ces 20 peptides, ils ont identifié le peptide correspondant à la séquence 131-151 (RIHMVYSKRSGKPRGYAFIEY) de la protéine 70K reconnu très précocement par les anticorps de ces souris (Monneaux et al, 2000). Ce peptide est capable de stimuler *in vitro* la prolifération et la sécrétion d'IL-2 des cellules T CD4+ purifiées à partir des ganglions.

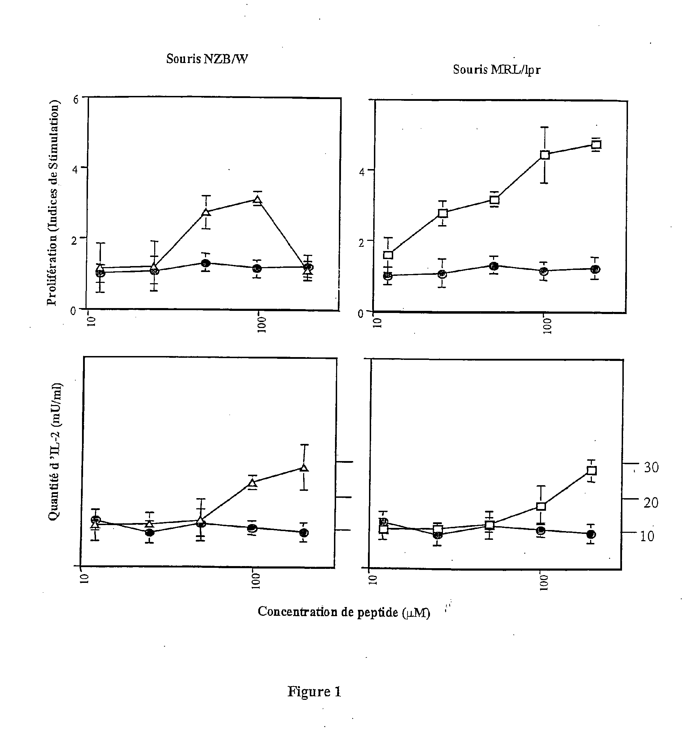

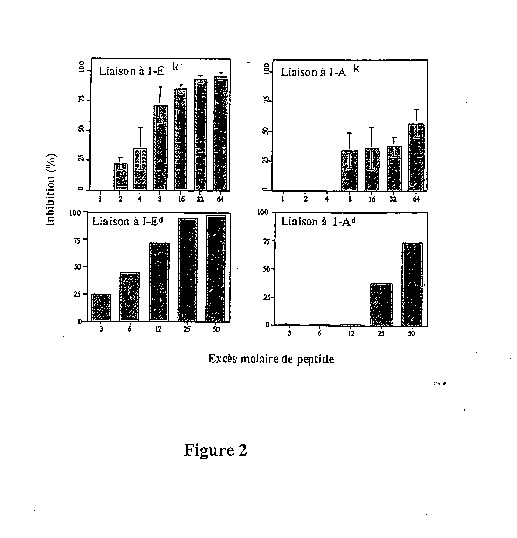

Par la suite, les Inventeurs ont montré que cette séquence représentait aussi un épitope majeur de la protéine 70K dans un autre modèle de souris lupique, la souris NZB/W (figure 1). Le peptide 131-151 est par ailleurs capable de se lier à diverses molécules de classe II du complexe majeur d'histocompatibilité (à la fois murines, et humaines; figure 2). Ce caractère universel constitue un avantage pour l'utilisation de cette séquence dans le développement de stratégies thérapeutiques dans le cadre du lupus érythémateux disséminé humain.

Les Inventeurs ont ensuite souhaité déterminer la nature exacte de la séquence de la protéine 70K capable d'activer les cellules T autoréactives. Les Inventeurs ont synthétisé plusieurs peptides correspondant aux formes phosphorylées et acétylées sur les résidus sérine et lysine du peptide 131-151, et étudié la capacité de ces peptides à être reconnus par les lymphocytes T et les anticorps de souris lupiques.

La présente invention découle de la mise en évidence par les Inventeurs que ces peptides phosphorylés et acétylés sont reconnus de façon égale voire supérieure au peptide parent non phosphorylé et non acétylé par les cellules T CD4 + et les anticorps de souris lupiques, et que l'administration de ces peptides phosphorylés et acétylés diminue la production de forts taux d'anticorps dirigés contre l'ADN, retarde l'apparition de la glomérulonéphrite et prolonge la survie des animaux, tandis que le peptide parent n'induit par contre aucun effet statistiquement significatif.

La présente invention a pour but de fournir de nouveaux peptides utilisables pour la préparation de médicaments dans le cadre du traitement de maladies auto-immunes, et plus particulièrement du lupus, présentant l'avantage d'être nettement plus efficaces que les peptides utilisés jusqu'à présent, et de ne pas présenter d'effets secondaires importants tels que ceux rencontrés avec les techniques actuelles de traitement, dans la mesure où les peptides modifiés de l'invention sont spécifiques des cellules délétères et ne ciblent que ces cellules, à la différence des immunosuppresseurs, cytokines, ou autres molécules utilisées actuellement qui agissent de manière globale sur le système immunitaire.

L'invention a pour objet l'utilisation de peptides comprenant des épitopes de protéines du soi des mammifères reconnus par des anticorps produits par le système immunitaire des mammifères atteints de pathologies auto-immunes, et, le cas échéant, par les cellules T auxiliaires desdits mammifères, lesdits épitopes étant tels que l'un au moins de leurs acides aminés comporte une modification de type post-traductionnel, pour la préparation d'un médicament destiné à la prévention ou au traitement desdites pathologies auto-immunes.

Par modification de type post-traductionnel, on entend dans ce qui précède et ce qui suit, tout type de modification des aminoacides d'une protéine donnée susceptible de se produire in vivo dans les cellules de l'organisme, tels que les processus de phosphorylation, d'acétylation, ou autre.

L'invention a plus particulièrement pour objet l'utilisation susmentionnée de peptides tels que définis ci-dessus, comprenant des épitopes dont l'un au moins de leurs acides aminés est modifié de manière à ce qu'il soit sous une forme phosphorylée, ou acétylée.

L'invention concerne plus particulièrement l'utilisation susmentionnée de peptides comprenant des épitopes issus des protéines d'origine humaine ou animale définies ci-dessus, lesdites protéines étant choisies parmi les nucléoprotéines, les protéines du nucléosome, du splicéosome, de la particule ribonucléoprotéique Ro, ou du ribosome par exemple.

L'invention concerne également l'utilisation de peptides tels que définis ci-dessus, pour la préparation d'un médicament destiné à la prévention ou au traitement :
- de pathologies auto-immunes de la famille des connectivites (maladies systémiques non spécifiques d'organes), telles que le lupus érythémateux disséminé (LED), la polyarthrite rhumatoïde, la connectivite mixte, le syndrome de Sjögren, ou l'arthrite chronique juvénile,
- ou de pathologies auto-immunes spécifiques d'organes, telles que la sclérose en plaques, le diabète insulino-dépendant, la maladie de Crohn, ou les maladies bulleuses.

L'invention a plus particulièrement pour objet l'utilisation de peptides tels que définis ci-dessus, pour la préparation d'un médicament destiné à la prévention ou au traitement du LED.

A ce titre, l'invention concerne plus particulièrement l'utilisation susmentionnée de peptides comprenant des épitopes issus de la protéine humaine ou murine U1-70K du splicéosome (décrite notamment dans Klein Gunnewiek et al, 1997).

L'invention a plus particulièrement pour objet encore l'utilisation susmentionnée de peptides comprenant la séquence délimitée par les acides aminés 131 et 151 de la protéine U1-70K humaine ou murine, et correspondant à la séquence SEQ ID NO : 1 suivante :
RIHMVYSKRSGKPRGYAFIEY
dans laquelle l'un au moins des acides aminés comporte une modification de type post-traductionnel, notamment dans laquelle l'un au moins des acides aminés est phosphorylé, ou acétylé.

L'invention concerne plus particulièrement l'utilisation susmentionnée de peptides comprenant la séquence SEQ ID NO : 1 dans laquelle l'un au moins des résidus sérine en position 7 ou 10 est phosphorylé, et/ou l'un au moins des résidus lysine en position 8 ou 12 est acétylé.

L'invention a plus particulièrement pour objet l'utilisation susmentionnée de peptides comprenant la séquence SEQ ID NO : 1 dans laquelle :
- la sérine en position 7 est phosphorylée,
- et/ou la sérine en position 10 est phosphorylée,
- et/ou la lysine en position 8 est acétylée,
- et/ou la lysine en position 12 est acétylée.

L'invention concerne plus particulièrement encore l'utilisation susmentionnée de peptides choisis parmi les suivants :
- la séquence SEQ ID NO : 1 dans laquelle la sérine en position 7 est phosphorylée,
- la séquence SEQ ID NO : 1 dans laquelle la sérine en position 7 ainsi que la sérine en position 10 sont phosphorylées,
- la séquence SEQ ID NO : 1 dans laquelle la sérine en position 7 est phosphorylée, et la lysine en position 8 est acétylée,
- la séquence SEQ ID NO : 1 dans laquelle la sérine en position 7 est phosphorylée, et la lysine en position 12 est acétylée,
- la séquence SEQ ID NO : 1 dans laquelle la sérine en position 7 est phosphorylée, et la lysine en position 8 ainsi que la lysine en position 12 sont acétylées,
- la séquence SEQ ID NO : 1 dans laquelle la sérine en position 7 ainsi que la sérine en position 10 sont phosphorylées, et la lysine en position 8 est acétylée,
- la séquence SEQ ID NO : 1 dans laquelle la sérine en position 7 ainsi que la sérine en position 10 sont phosphorylées, et la lysine en position 12 est acétylée,
- la séquence SEQ ID NO : 1 dans laquelle la sérine en position 7 ainsi que la sérine en position 10 sont phosphorylées, et la lysine en position 8 ainsi que la lysine en position 12 sont acétylées,
- la séquence SEQ ID NO : 1 dans laquelle la sérine en position 10 est phosphorylée,
- la séquence SEQ ID NO : 1 dans laquelle la sérine en position 10 est phosphorylée, et la lysine en position 8 est acétylée,
- la séquence SEQ ID NO : 1 dans laquelle la sérine en position 10 est phosphorylée, et la lysine en position 12 est acétylée,
- la séquence SEQ ID NO : 1 dans laquelle la sérine en position 10 est phosphorylée, et la lysine en position 8 ainsi que la lysine en position 12 sont acétylées,
- la séquence SEQ ID NO : 1 dans laquelle la lysine en position 8 est acétylée,
- la séquence SEQ ID NO : 1 dans laquelle la lysine en position 12 est acétylée,
- la séquence SEQ ID NO : 1 dans laquelle la lysine en position 8 et la lysine en position 12 sont acétylées.

L'invention a plus particulièrement pour objet encore l'utilisation susmentionnée de peptides choisis parmi les suivants :
- la séquence SEQ ID NO : 1 dans laquelle la sérine en position 7 est phosphorylée,
- la séquence SEQ ID NO : 1 dans laquelle la sérine en position 10 est phosphorylée,
- la séquence SEQ ID NO : 1 dans laquelle la lysine en position 8 est acétylée,
- la séquence SEQ ID NO : 1 dans laquelle la lysine en position 12 est acétylée,
- la séquence SEQ ID NO : 1 dans laquelle la lysine en position 8 et la lysine en position 12 sont acétylées.

L'invention a également pour objet toute composition pharmaceutique caractérisée en ce qu'elle comprend au moins un peptide choisi parmi ceux définis ci-dessus, en association avec un véhicule pharmaceutiquement acceptable.

L'invention a plus particulièrement pour objet toute composition pharmaceutique telle que définie ci-dessus, caractérisée en ce qu'elle comprend au moins un peptide choisi parmi ceux comprenant la séquence SEQ ID NO : 1 dans laquelle l'un au moins des acides aminés comporte une modification de type post-traductionnel, notamment par phosphorylation, ou acétylation.

L'invention concerne plus particulièrement toute composition pharmaceutique telle que définie ci-dessus, caractérisée en ce qu'elle comprend au moins un peptide choisi parmi ceux comprenant la séquence SEQ ID NO : 1 dans laquelle l'un au moins des résidus sérine en position 7 ou 10 est phosphorylé, et/ou l'un au moins des résidus lysine en position 8 ou 12 est acétylé.

L'invention a plus particulièrement pour objet toute composition pharmaceutique telle que définie ci-dessus, caractérisée en ce qu'elle comprend au moins un peptide choisi parmi ceux comprenant la séquence SEQ ID NO : 1 dans laquelle :
- la sérine en position 7 est phosphorylée,
- et/ou la sérine en position 10 est phosphorylée,
- et/ou la lysine en position 8 est acétylée,
- et/ou la lysine en position 12 est acétylée.

L'invention concerne plus particulièrement encore toute composition pharmaceutique telle que définie ci-dessus, caractérisée en ce qu'elle comprend au moins un peptide choisi parmi les suivants :
- la séquence SEQ ID NO : 1 dans laquelle la sérine en position 7 est phosphorylée,
- la séquence SEQ ID NO : 1 dans laquelle la sérine en position 7 ainsi que la sérine en position 10 sont phosphorylées,
- la séquence SEQ ID NO : 1 dans laquelle la sérine en position 7 est phosphorylée, et la lysine en position 8 est acétylée,
- la séquence SEQ ID NO : 1 dans laquelle la sérine en position 7 est phosphorylée, et la lysine en position 12 est acétylée,
- la séquence SEQ ID NO : 1 dans laquelle la sérine en position 7 est phosphorylée, et la lysine en position 8 ainsi que la lysine en position 12 sont acétylées,
- la séquence SEQ ID NO : 1 dans laquelle la sérine en position 7 ainsi que la sérine en position 10 sont phosphorylées, et la lysine en position 8 est acétylée,
- la séquence SEQ ID NO : 1 dans laquelle la sérine en position 7 ainsi que la sérine en position 10 sont phosphorylées, et la lysine en position 12 est acétylée,
- la séquence SEQ ID NO : 1 dans laquelle la sérine en position 7 ainsi que la sérine en position 10 sont phosphorylées, et la lysine en position 8 ainsi que la lysine en position 12 sont acétylées,
- la séquence SEQ ID NO : 1 dans laquelle la sérine en position 10 est phosphorylée,
- la séquence SEQ ID NO : 1 dans laquelle la sérine en position 10 est phosphorylée, et la lysine en position 8 est acétylée,
- la séquence SEQ ID NO : 1 dans laquelle la sérine en position 10 est phosphorylée, et la lysine en position 12 est acétylée,
- la séquence SEQ ID NO : 1 dans laquelle la sérine en position 10 est phosphorylée, et la lysine en position 8 ainsi que la lysine en position 12 sont acétylées,
- la séquence SEQ ID NO : 1 dans laquelle la lysine en position 8 est acétylée,
- la séquence SEQ ID NO : 1 dans laquelle la lysine en position 12 est acétylée,
- la séquence SEQ ID NO : 1 dans laquelle la lysine en position 8 et la lysine en position 12 sont acétylées.

L'invention a plus particulièrement pour objet encore toute composition pharmaceutique telle que définie ci-dessus, caractérisée en ce qu'elle comprend au moins un peptide choisi parmi les suivants :
- la séquence SEQ ID NO : 1 dans laquelle la sérine en position 7 est phosphorylée,
- la séquence SEQ ID NO : 1 dans laquelle la sérine en position 10 est phosphorylée,
- la séquence SEQ ID NO : 1 dans laquelle la lysine en position 8 est acétylée,
- la séquence SEQ ID NO : 1 dans laquelle la lysine en position 12 est acétylée,
- la séquence SEQ ID NO : 1 dans laquelle la lysine en position 8 et la lysine en position 12 sont acétylées.

Avantageusement, les compositions pharmaceutiques susmentionnées de l'invention, sont caractérisées en ce qu'elles se présentent sous une forme administrable par voie systémique (à savoir par voie intraveineuse, intramusculaire, intrapéritonéale, sous-cutanée), ou non invasive (par exemple intranasale, orale, ou épicutanée).

Avantageusement encore, les compositions pharmaceutiques susmentionnées de l'invention, sont caractérisées en ce que les doses journalières de peptides chez l'homme sont d'environ 100 ng à environ 5 mg.

L'invention concerne également les peptides comprenant la séquence délimitée par les acides aminés 131 et 151 de la protéine U1-70K humaine, ou murine, et correspondant à la séquence SEQ ID NO : 1 suivante :
RIHMVYSKRSGKPRGYAFIEY
dans laquelle l'un au moins des acides aminés est phosphorylé, ou acétylé.

L'invention a plus particulièrement pour objet les peptides susmentionnés, comprenant la séquence SEQ ID NO : 1 dans laquelle l'un au moins des résidus sérine en position 7 ou 10 est phosphorylé, et/ou l'un au moins des résidus lysine en position 8 ou 12 est acétylé.

L'invention concerne plus particulièrement les peptides susmentionnés, comprenant la séquence SEQ ID NO : 1 dans laquelle :
- la sérine en position 7 est phosphorylée,
- et/ou la sérine en position 10 est phosphorylée,
- et/ou la lysine en position 8 est acétylée,
- et/ou la lysine en position 12 est acétylée.

L'invention concerne plus particulièrement encore les peptides suivants :
- la séquence SEQ ID NO : 1 dans laquelle la sérine en position 7 est phosphorylée,
- la séquence SEQ ID NO : 1 dans laquelle la sérine en position 7 ainsi que la sérine en position 10 sont phosphorylées,
- la séquence SEQ ID NO : 1 dans laquelle la sérine en position 7 est phosphorylée, et la lysine en position 8 est acétylée,
- la séquence SEQ ID NO : 1 dans laquelle la sérine en position 7 est phosphorylée, et la lysine en position 12 est acétylée,
- la séquence SEQ ID NO : 1 dans laquelle la sérine en position 7 est phosphorylée, et la lysine en position 8 ainsi que la lysine en position 12 sont acétylées,
- la séquence SEQ ID NO : 1 dans laquelle la sérine en position 7 ainsi que la sérine en position 10 sont phosphorylées, et la lysine en position 8 est acétylée,
- la séquence SEQ ID NO : 1 dans laquelle la sérine en position 7 ainsi que la sérine en position 10 sont phosphorylées, et la lysine en position 12 est acétylée,
- la séquence SEQ ID NO : 1 dans laquelle la sérine en position 7 ainsi que la sérine en position 10 sont phosphorylées, et la lysine en position 8 ainsi que la lysine en position 12 sont acétylées,
- la séquence SEQ ID NO : 1 dans laquelle la sérine en position 10 est phosphorylée,
- la séquence SEQ ID NO : 1 dans laquelle la sérine en position 10 est phosphorylée, et la lysine en position 8 est acétylée,
- la séquence SEQ ID NO : 1 dans laquelle la sérine en position 10 est phosphorylée, et la lysine en position 12 est acétylée,
- la séquence SEQ ID NO : 1 dans laquelle la sérine en position 10 est phosphorylée, et la lysine en position 8 ainsi que la lysine en position 12 sont acétylées,
- la séquence SEQ ID NO : 1 dans laquelle la lysine en position 8 est acétylée,
- la séquence SEQ ID NO : 1 dans laquelle la lysine en position 12 est acétylée,
- la séquence SEQ ID NO : 1 dans laquelle la lysine en position 8 et la lysine en position 12 sont acétylées.

L'invention a plus particulièrement pour objet encore les peptides suivants :
- la séquence SEQ ID NO : 1 dans laquelle la sérine en position 7 est phosphorylée,
- la séquence SEQ ID NO : 1 dans laquelle la sérine en position 10 est phosphorylée,
- la séquence SEQ ID NO : 1 dans laquelle la lysine en position 8 est acétylée,
- la séquence SEQ ID NO : 1 dans laquelle la lysine en position 12 est acétylée,
- la séquence SEQ ID NO : 1 dans laquelle la lysine en position 8 et la lysine en position 12 sont acétylées.

L'invention sera davantage illustrée à l'aide de la description détaillée qui suit de la synthèse des peptides modifiés de l'invention, ainsi que de l'étude de leurs propriétés biologiques.

### I) Synthèses

Les peptides phosphorylés P137 (correspondant à la séquence SEQ ID NO : 1 dans laquelle la sérine en position 7 est phosphorylée), et P140 (correspondant à la séquence SEQ ID NO : 1 dans laquelle la sérine en position 10 est phosphorylée), et les peptides acétylés Ac138 (correspondant à la séquence SEQ ID NO : 1 dans laquelle la lysine en position 8 est acétylée), Ac142 (correspondant à la séquence SEQ ID NO : 1 dans laquelle la lysine en position 12 est acétylée), et Ac138+142 (correspondant à la séquence SEQ ID NO : 1 dans laquelle la lysine en position 8 et celle en position 12 sont acétylées), ainsi que le peptide brouillé Sc : YVSRYFGSAIRHEPKMKIYRG, et le peptide brouillé ScP correspondant à Sc dans lequel la sérine en position 8 est phosphorylée, (utilisés comme contrôles négatifs dans les tests qui suivent) correspondant respectivement à la séquence SEQ ID NO : 1 et à la séquence de P140 dans lesquelles les acides aminés sont dans un ordre différent et aléatoire, ont été synthétisés chimiquement en phase solide sur un synthétiseur automatique en utilisant la stratégie Fmoc (N-(9-fluorenyl)methoxycarbonyl). Afin d'introduire les résidus de sérine phosphorylée à la place des résidus de sérine ou les résidus de lysine acétylée à la place des résidus de lysine, un dérivé de la sérine de type Fmoc-Ser(PO(Obz)OH)-OH, ou un dérivé de lysine de type Fmoc-Lys(Ac), ont été utilisés. Le temps de couplage est augmenté à 30 minutes et un second couplage est systématiquement effectué. Après le clivage en milieu acide, chaque peptide est précipité par de l'éther froid, solubilisé dans une solution d'eau et d'acétonitrile et enfin lyophilisé. Les peptides sont ensuite purifiés par RP-HPLC, leur intégrité et leur pureté ont été analysées par HPLC analytique et par spectrométrie de masse (Maldi-TOF).

| | Pureté | Masse attendue | Masse mesurée |
|---|---|---|---|
| P137 | 71.1% | 2639 | 2637.04 |
| P140 | 90.2% | 2639 | 2637.03 |
| Ac138 | 88.8% | 2600 | 2602.3 |
| Ac142 | 83.4% | 2600 | 2600.3 |
| Ac138+142 | 85.1% | 2643 | 2644.68 |
| Sc | 96.5% | 2558 | 2559.56 |
| ScP | 97.2% | 2637 | 2637.06 |

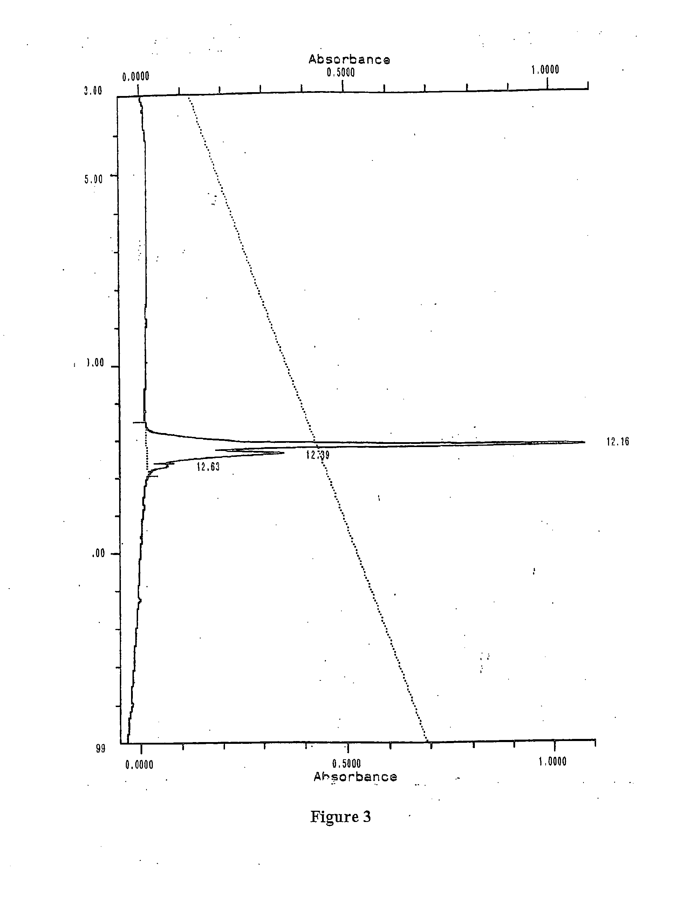

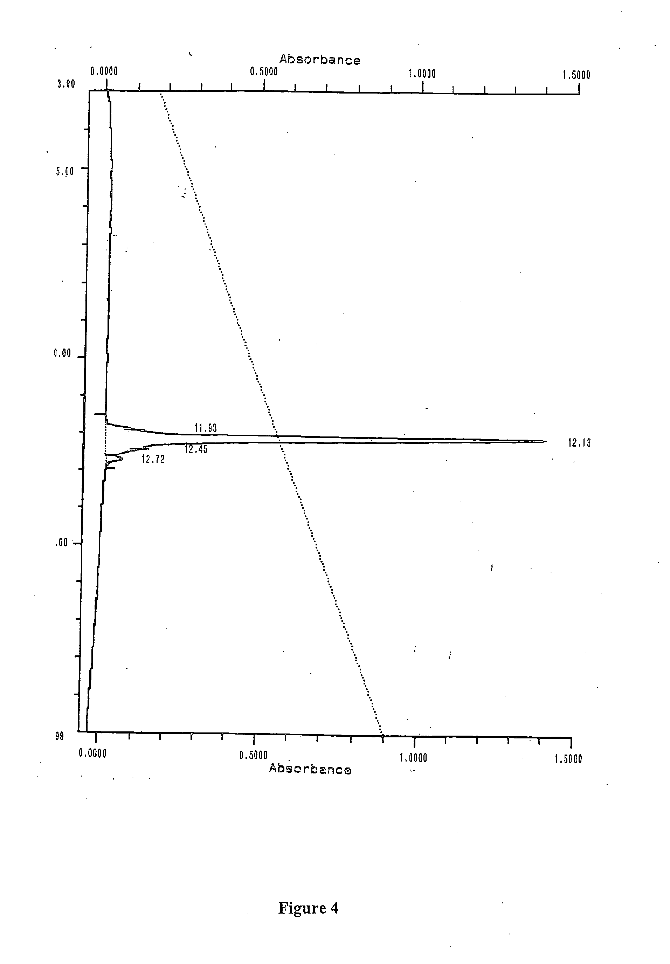

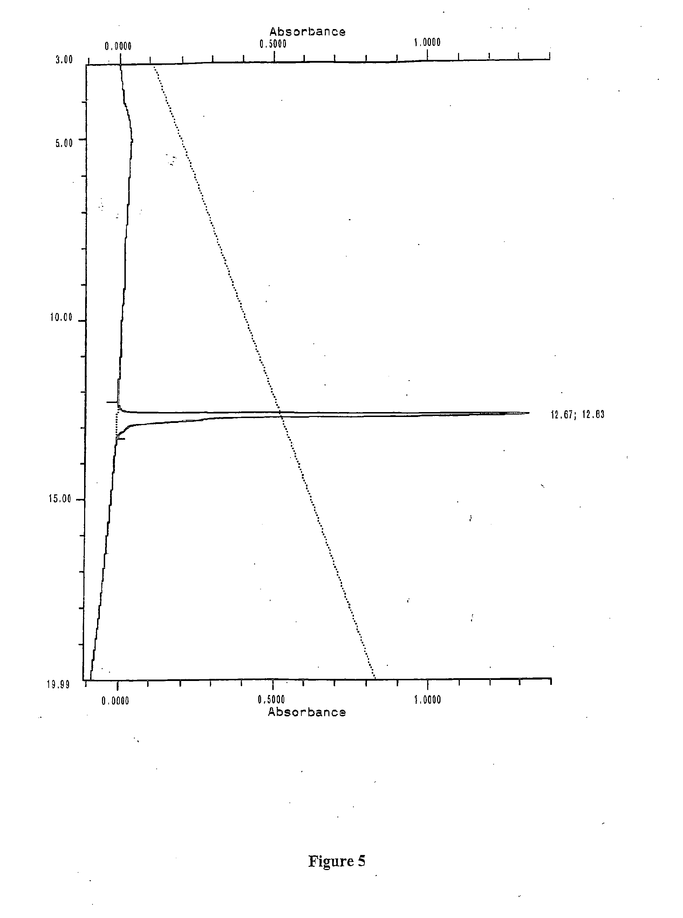

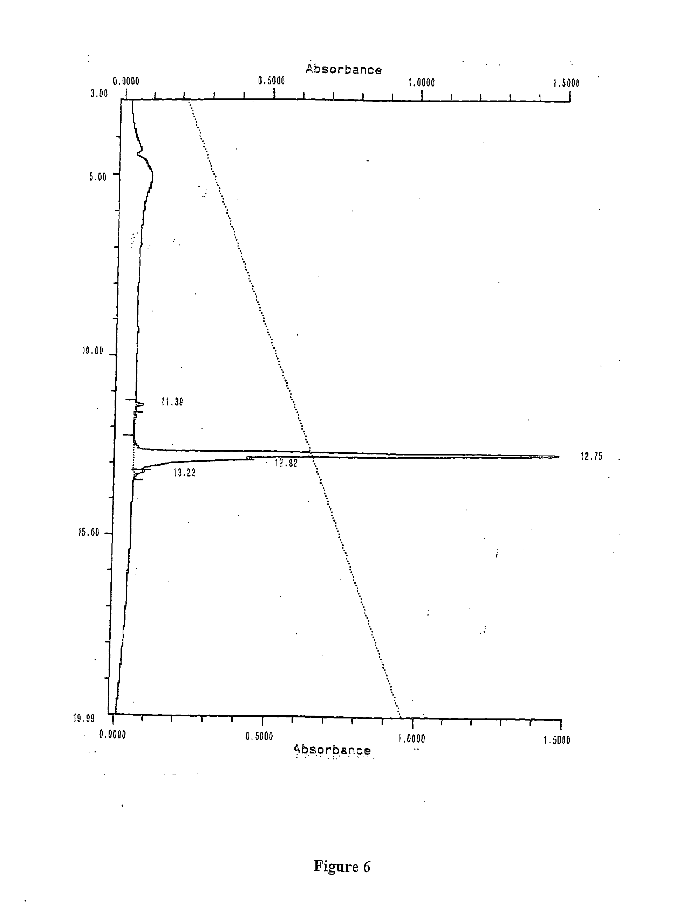

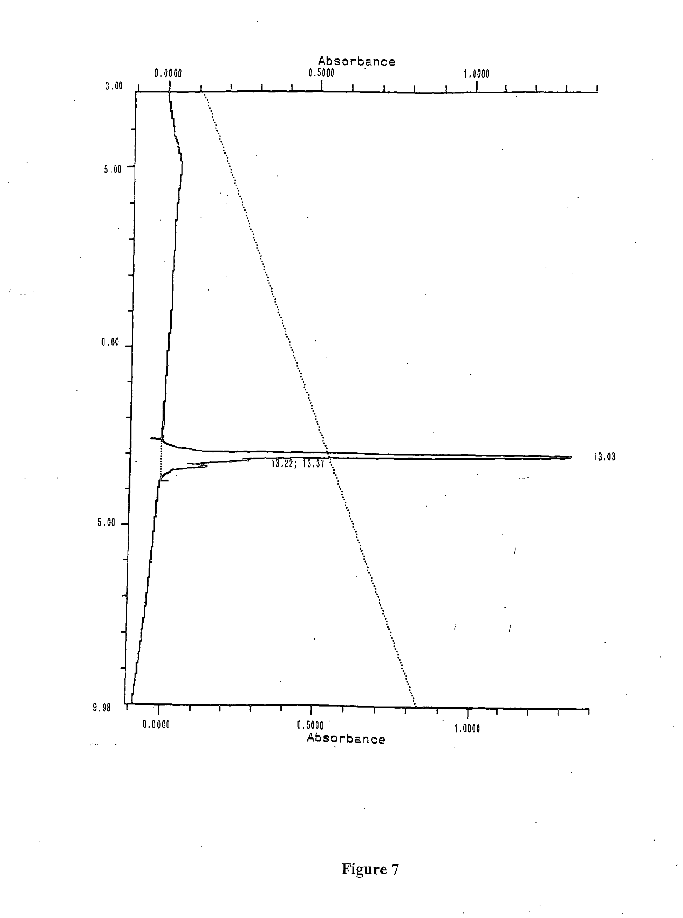

Les profils HPLC des peptides P137, P140, Ac138, Ac142, Ac138+142, Sc et ScP sont représentés respectivement sur les figures 3, 4, 5, 6, 7, 15 et 16 (matériel utilisé : colonne Nucifosil C 1B 150x4.6 mm; débit : 1.2 ml/mn; détection UV : 210 nm; gradient utilisé : 5-65 en 20 min en eau + TFA 0,1 % et acétonitrile + TFA 0,08%).

Les résultats sont indiqués dans les tableaux A, B, C, D, E, F et G ci-après correspondant respectivement aux profils HPLC des peptides P137, P140, Ac 138, Ac 142, Ac 138 + 142, Sc et ScP.

**Tableau A**

| nombre de pics | temps de rétention | zone de pic | pourcentage de zone |
|---|---|---|---|
| 1 | 12.16 | 164.7 | 71.1 |
| 2 | 12.39 | 60.5 | 26.1 |
| 3 | 12.63 | 6.6 | 2.3 |
| Total | | 231.8 | 100.0 |

**Tableau B**

| nombre de pics | temps de rétention | zone de pic | pourcentage de zone |
|---|---|---|---|
| 1 | 11.93 | 8.9 | 3.1 |
| 2 | 12.13 | 254.4 | 90.2 |
| 3 | 12.45 | 11.5 | 4.1 |
| 4 | 12.72 | 7.3 | 2.6 |
| Total | | 292.1 | 100.0 |

**Tableau C**

| nombre de pics | temps de rétention | zone de pic | pourcentage de zone |
|---|---|---|---|
| 1 | 12.67 | 171.6 | 88.8 |
| 2 | 12.83 | 21.7 | 11.2 |
| Total | | 193.3 | 100.0 |

**Tableau D**

| nombre de pics | temps de rétention | zone de pic | pourcentage de zone |
|---|---|---|---|
| 1 | 11.39 | 1.9 | 0.8 |
| 2 | 12.75 | 203.1 | 83.4 |
| 3 | 12.92 | 34.8 | 14.2 |
| 4 | 13.22 | 3.8 | 1.6 |
| Total | | 243.6 | 100.0 |

**Tableau E**

| nombre de pics | temps de rétention | zone de pic | pourcentage de zone |
|---|---|---|---|
| 1 | 13.03 | 213.3 | 85.1 |
| 2 | 13.22 | 15.2 | 6.1 |
| 3 | 13.37 | 22.1 | 8.8 |
| Total | | 250.6 | 100.0 |

**Tableau F**

| nombre de pics | temps de rétention | zone de pic | pourcentage de zone |
|---|---|---|---|
| 1 | 12.08 | 219.3 | 96.5 |
| 2 | 12.67 | 2.5 | 1.1 |
| 3 | 12.63 | 5.5 | 2.4 |
| Total | | 227.2 | 100.0 |

**Tableau G**

| nombre de pics | temps de rétention | zone de pic | pourcentage de zone |
|---|---|---|---|
| 1 | 12.39 | 273.4 | 97.2 |
| 2 | 12.93 | 2.4 | 0.9 |
| 3 | 13.63 | 5.4 | 1.9 |
| Total | | 281.2 | 100.0 |

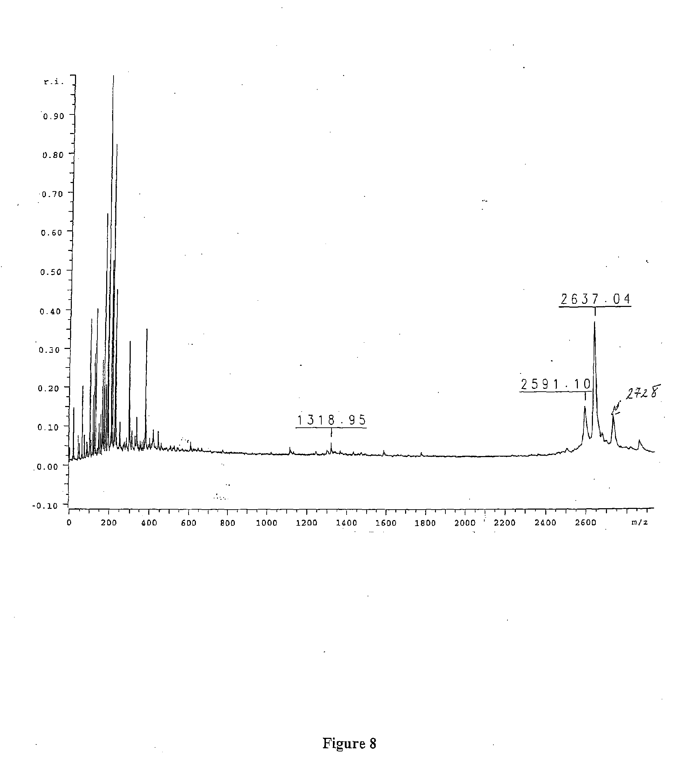

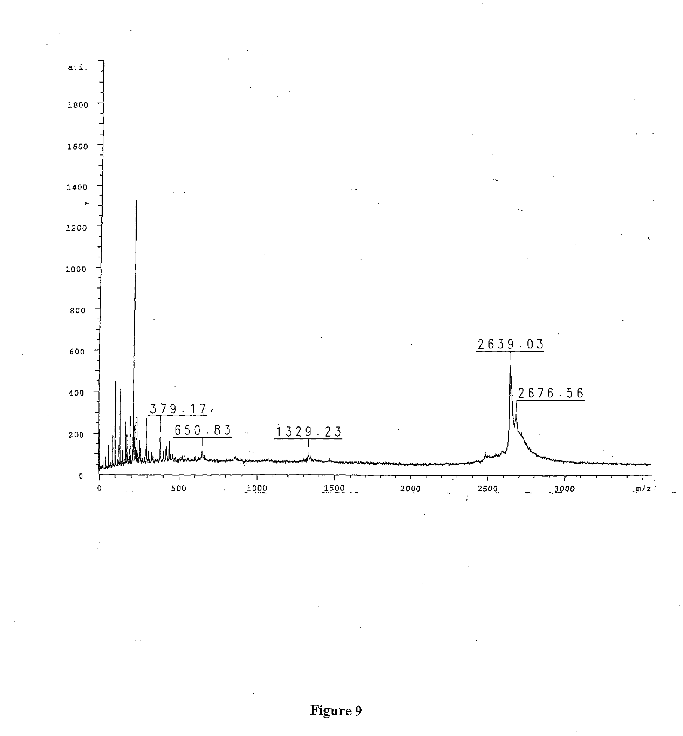

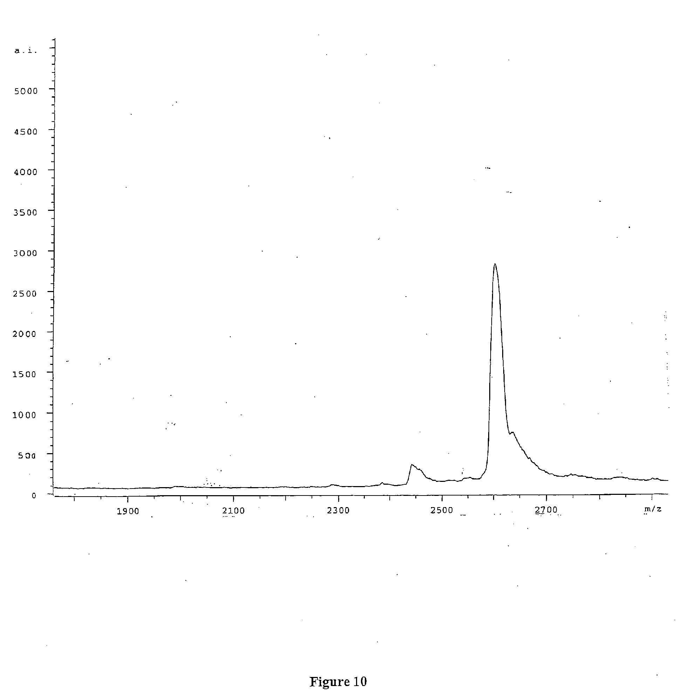

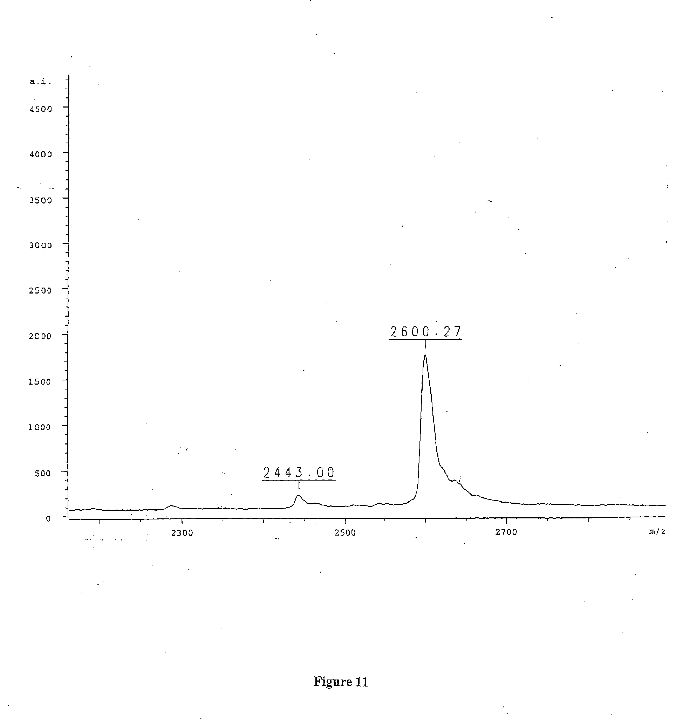

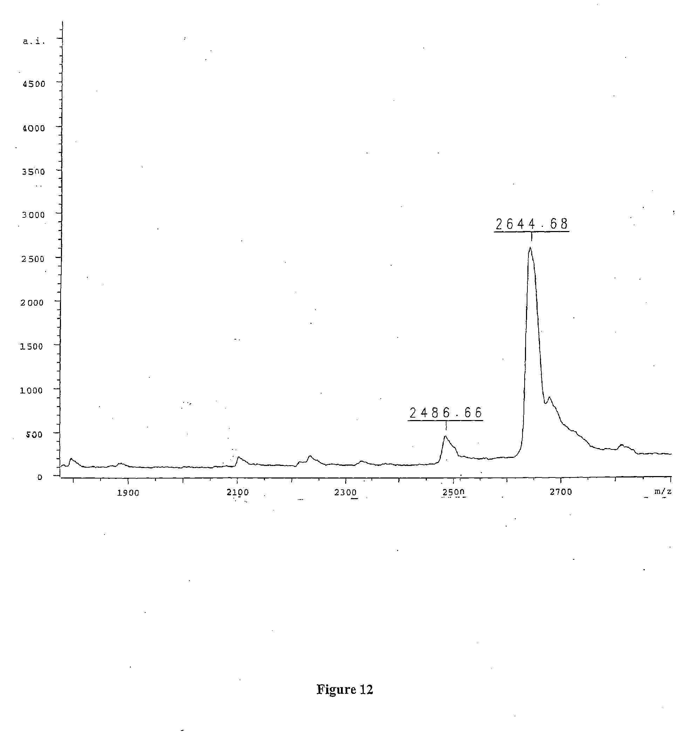

Les spectres de masse des peptides P137, P140, Ac 138, Ac 142, Ac 138+142, Sc et ScP sont représentés respectivement sur les figures 8, 9, 10, 11, 12, 17 et 18.

### II) Propriétés biologiques

La protéine 70K étant fortement phosphorylée in vivo (Woppmann et al, 1993), et bien que le nombre de sites phosphorylés et leur identité ne soient pas connus, les Inventeurs ont synthétisé plusieurs peptides correspondant aux formes phosphorylées et acétylées respectivement sur les résidus sérine et lysine du peptide 131-151, et étudié la capacité de ces peptides à être reconnus par les lymphocytes T et les anticorps de souris lupiques.

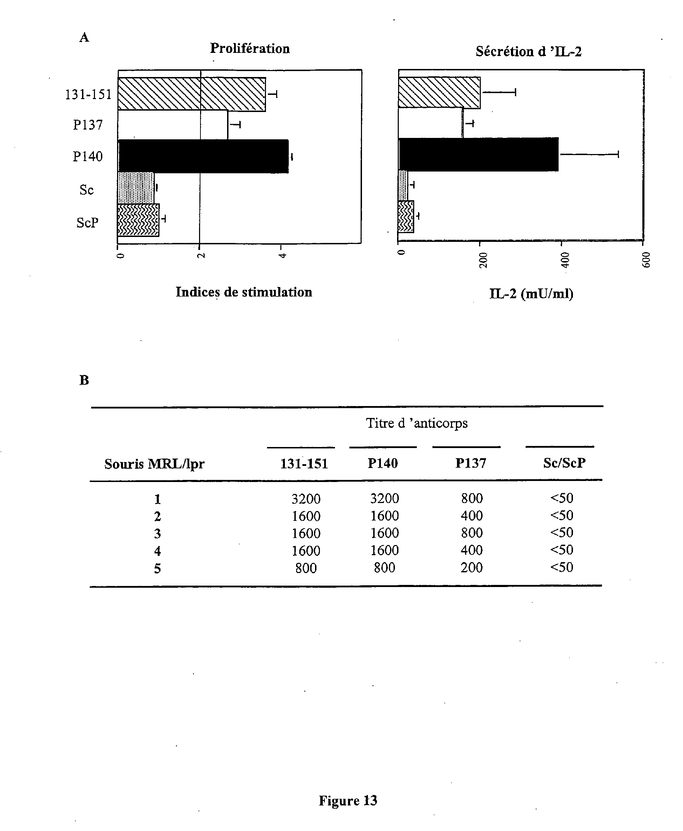

Les Inventeurs démontrent dans le cadre de la présente invention que le peptide phosphorylé en position 140 est reconnu de façon égale voire supérieure au peptide non phosphorylé par les cellules T CD4 + et les anticorps de souris lupiques (figure 13). Les deux peptides (phosphorylé en position 140 et non phosphorylé) ont été utilisés pour une étude de restauration de tolérance au soi. Ces deux peptides ont été injectés par voie intraveineuse et voie intra-nasale à des souris pré-autoimmunes, et l'évolution de la maladie chez ces souris a été suivie.

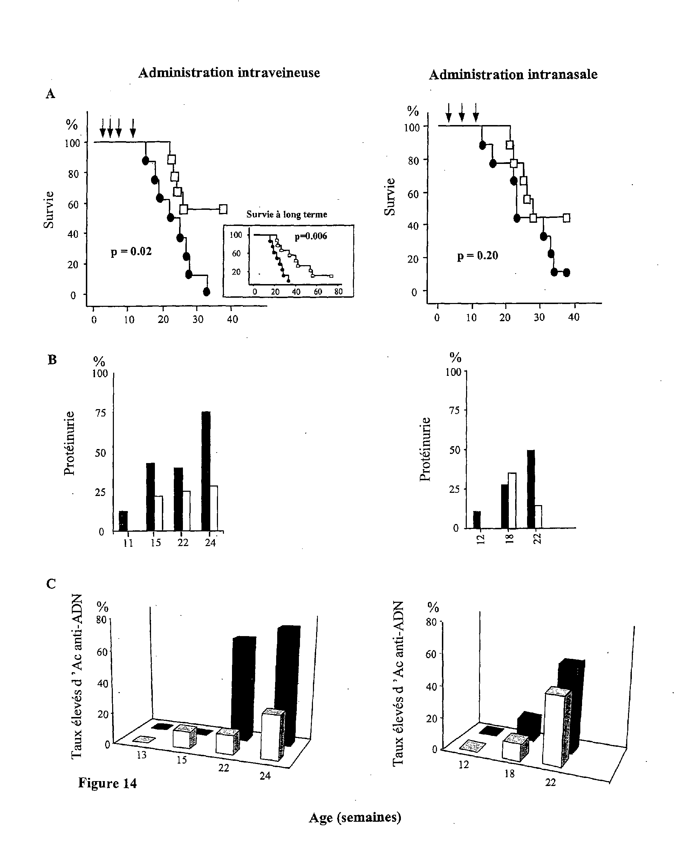

Les Inventeurs ont mis en évidence que l'administration par voie intraveineuse mais pas par voie intra-nasale du peptide phosphorylé P140 diminue la production de forts taux d'anticorps dirigés contre l'ADN, retarde l'apparition de la glomérulonéphrite et prolonge la survie des animaux (figure 14), tandis que le peptide parent n'induit par contre aucun effet statistiquement significatif.

De plus, des études avec 3 peptides acétylés sur les lysines 138, 142 et 138 + 142 ont été effectuées. Comme dans le cas des peptides phosphorylés, rien ne permet d'affirmer que ces positions sont réellement acétylées *in vivo.* Les premiers résultats ont montré que :
- les 3 peptides acétylés sont au moins aussi bien voire mieux reconnus que le peptide parent par les cellules T CD4 + de souris normales immunisées contre le peptide non modifié : les taux de prolifération sont supérieurs, les taux de sécrétion d'IL2 sont équivalents et les taux de production d'interféron y sont supérieurs,
- les 3 peptides acétylés sont au moins aussi bien voire mieux reconnus que le peptide parent par les cellules T CD4+ de souris autoimmunes : les taux de prolifération sont supérieurs,
- les 3 peptides acétylés sont reconnus par les anticorps de souris dirigés contre le peptide parent.

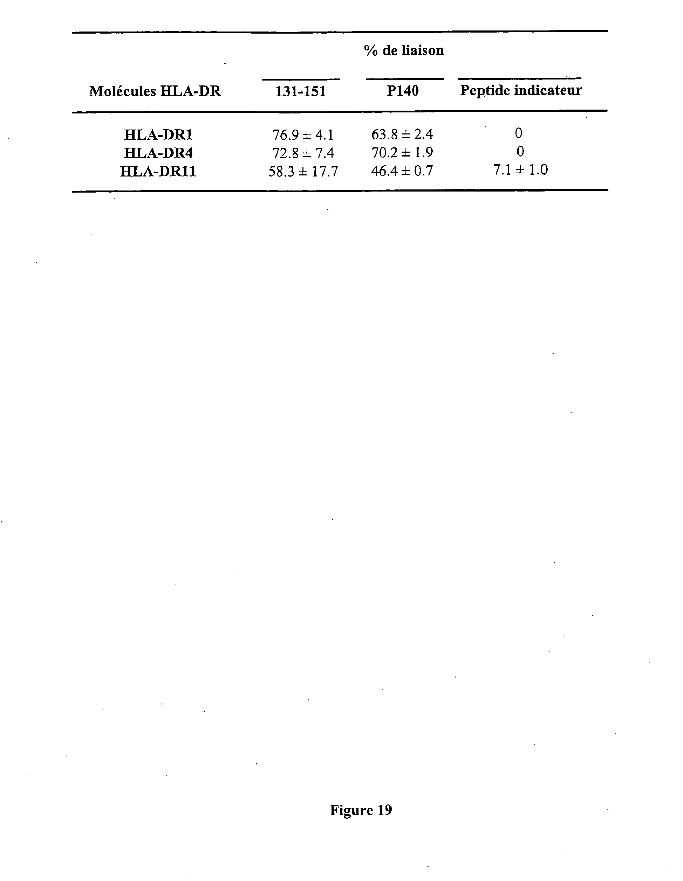

Enfin les Inventeurs ont démontré que les peptides 131-151 et P140 étaient capables de lier diverses molécules du CMH de classe II humaines (HLA-DR1, -DR4 et -DR11) (Figure 19).

### Références bibliographiques

Andersen M.H., Bonfill J.E., Neisig A., Arsequell G., Sondergaard I., Valencia G., Neefjes J., Zeuthen J., Elliot T. and Haurum J. S. (1999) Phosphorylated peptides can be transported by TAP molecules, presented by class I MHC molecules, and recognized by phosphorylated-specific CTL. J. Immunol. 163:3812-3818.
Eilat E., Zinger H., Nyska A. and Mozes E. (2000) Prevention of systemic lupus erythematosus-like disease in (NZBxNZW)F1 mice by treating with CDR1- and CDR3-based peptides of a pathogenic autoantibody. J. Clin. Immunol. 20 :268-278.
Jouanne C., Avrameas S. and Payelle-Brogard B. (1999) A peptide derived from a polyreactive monoclonal anti-DNA natural antibody modulate lupus development in (NZBxNZW)F1 mice. Immunology 96:333-339.
Kaliyaperumal A., Michaels M.A. ans Datta S.K. (1999) Antigen-specific therapy of murine lupus nephritis using nucleosomal peptides: tolérance spreading impairs pathogenic function of autoimmune T and B cells. J. Immunol. 162:5775-5783.
Klein Gunnewiek, J. M. T. Van De Putte, L.B.A. and van Venrooij, W. J., The U1 snRNP complex : an autoantigen in connective tissue disease. Clin. Exp. Rheumatol. 1997, 15 : 549-560.
Marino M., Ruvo M., de Fales S. and Facsina G. (2000) Prevention of systemic lupus erythematosus in MRL/lpr mice by administration of an immunoglobulin-binding peptide. Nature Biotechn. 18 : 735-739.
Monneaux F., Briand J.-P. and Muller S. (2000) B and T cell immune response to snRNP in lupus mice. Autoreactive CD4 + T cells recognize a T cell epitope located within the conserved RNP consensus sequence of the 70K protein. Eur. J. Immunol. 20 :2191-2200.
Monneaux F. and Muller S. (2000) Laboratory protocols for the identification of Th cell epitopes on self antigens in mice with systemic autoimmune diseases. J. Immunol. Meth. 244 :195-204.
Singh R.R., Ebling F.M., Sercarz E.E. and Hahn B.H. (1995) Immune tolérance to autoantibody-derived peptides delays development of autoimmunity in murine lupus. J. Clin. Invest. 96:2990-2996.
Utz, P. J., and P. Anderson. (1998). Posttranslational protein modifications, apoptosis, ans the bypass of tolérance to autoantigens, Arthritis Rheum 41 : 1152-1160.
Woppmann, A. C. L. Will. U. Kornstadt, P. Zuo, J. L. Manley, and R. Lurhmann, (1993). Identification of an snRNP-associated kinase activity that phosphorylatres arginine/serine rich domains typical of slicing factors. Nucleic Acids Res 21 : 2815-2822.
Zarling A. L., Ficarro S.B., Shabanowitz J., Hunt D. F. and Engelhard V.H. (2000) Phosphorylated peptides are naturally processed and presented by major histocompatibility complex class 1 molecules in vivo. J. Exp. Med. 192:1755-1762.

### Légende des figures

- Figure 1 : reconnaissance du peptide 131-151 de la protéine 70K par les cellules T CD4+ de souris lupiques MRL/lpr (colonne de droite) ou NZB/W (colonne de gauche); les concentrations en peptide sont indiquées en abscisse, la prolifération des cellules T CD4+ de souris MRL/lpr et BW, représentée en ordonnées des graphes de la première ligne, est mesurée ex vivo en présence des différentes concentrations de peptide 131-151 de la protéine 70K; la prolifération est exprimée en indices de stimulation correspondant à la radioactivité incorporée dans l'ADN des cellules (en coups par minutes) en présence de peptide sur l'incorporation de radioactivité en absence de peptide; la sécrétion d'IL-2, représentée en ordonnées des graphes de la deuxième ligne, est mesurée dans les surnageants après 24h de culture par un test biologique; la concentration en IL-2 est déterminée grâce à une gamme étalon d'IL-2 recombinante et est exprimée en mU/ml.
- Figure 2 : liaison du peptide 131-151 aux molécules du complexe majeur d'histocompatibilité de clase II murines (I-E^{k}, I-A^{k}, I-E^{d}, I-A^{d}); des fibroblastes transfectés par les molécules I-E^{k}, ou I-A^{k}, ou I-E^{d}, ou I-A^{d} sont pré-incubés avec différentes concentrations du peptide 131-151; après 30 min à 37°C, les peptides analogues 12-26CI ou 16-33 du récepteur #2-adrénergique, ainsi que les hybridomes T respectifs qui reconnaissent ces peptides dans le contexte adapté (81 pour I-E^{k}, E7E9 pour I-A^{k}, 26.1 pour I-E^{d}, et 26.2 pour I-A^{d}) sont ajoutés; les surnageants sont récupérés après 24 h de culture et la sécrétion d'IL-2 est évaluée comme précédemment; les résultats sont exprimés en % d'inhibition représentant la capacité du peptide 131-151 à inhiber la liaison des peptides analogues 12-26 CI et 16-33β2 aux molécules des classes II.
- Figure 3 : profil HPLC du peptide P137.
- Figure 4 : profil HPLC du peptide P140.
- Figure 5 : profil HPLC du peptide Ac 138.
- Figure 6 : profil HPLC du peptide Ac 142.
- Figure 7 : profil HPLC du peptide Ac138 + 142.
- Figure 8 : spectre de masse du peptide P137.
- Figure 9 : spectre de masse du peptide P140.
- Figure 10 : spectre de masse du peptide Ac 138.
- Figure 11 : spectre de masse du peptide Ac 142.
- Figure 12 : spectre de masse du peptide Ac 138+142.
- Figure 13 : reconnaissance du peptide P140
   - par les lymphocytes T CD4⁺ (A) de souris lupiques MRL/lpr ; le graphe de gauche représente la prolifération des cellules T CD4⁺ exprimée en indices de stimulation tels que définis ci-dessus en présence du peptide 131-151, des peptides phosphorylés P137 et P140 et de deux peptides brouillés phosphorylés ou non (Sc et ScP) ; la limite de positivité correspond à 2.0 ; le graphe de droite représente la sécrétion d'IL-2 (mU/ml) en présence du peptide 131-151, des peptides P137 et P140 et des peptides Sc et ScP.
   - et par les anticorps (B) de souris lupiques MRL/lpr ; les résultats sont exprimés en titre d'anticorps correspondant à la dilution permettant d'obtenir en test ELISA une valeur de DO à 450nm égale à 0.2.
- Figure 14 : effet de l'administration à des souris pré-autoimmunes MRL/lpr de la forme phosphorylée du peptide 131-151 de la protéine 70K (peptide P140) ; le graphe de gauche représente les groupes de souris qui ont reçu le peptide ou la solution saline par voie intraveineuse et le graphe de droite représente les souris qui ont reçu le peptide ou la solution saline par voie intranasale ; les résultats sont exprimés en pourcentage de survie en fonction de l'âge en semaines des souris lupiques utilisées (A), en pourcentage de protéinurie positive en fonction de l'âge en semaines des souris lupiques utilisées (B) et en pourcentage de taux élevés d'anticorps dirigés contre l'ADN en fonction de l'âge des souris lupiques utilisées (C) ; les dates d'administration sont représentées par des flèches, les symboles et barres vides correspondent aux souris ayant reçu le peptide P140 ; le groupe contrôle représenté par les symboles et barres pleins n'a reçu que la solution saline : dans le groupe injecté avec le peptide 131-151 nominal non phosphorylé, 25 % des souris ont survécu à 35 semaines (p=0.2 par rapport aux souris contrôles), et la protéinurie était à peine diminuée.

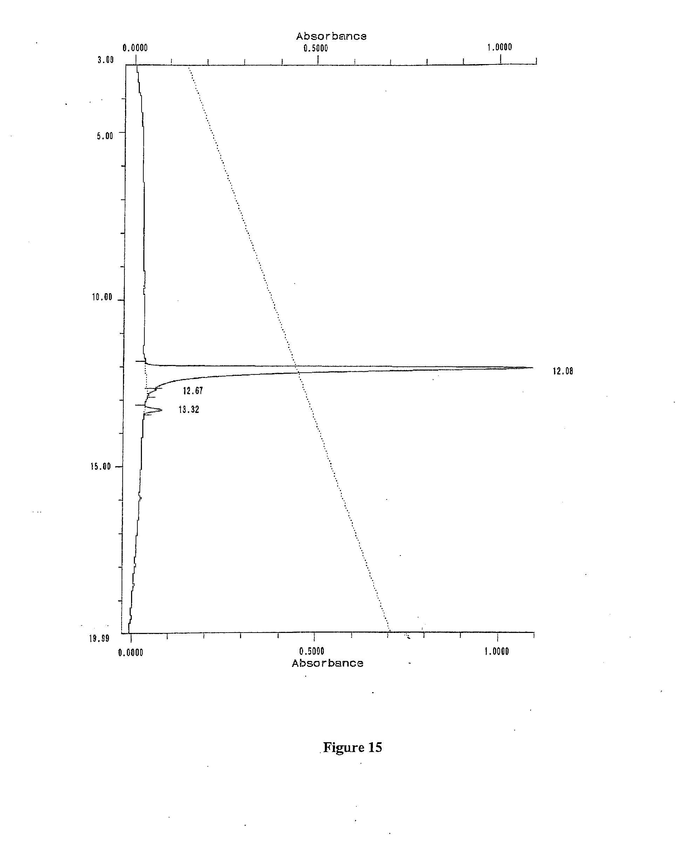

- Figure 15 : profil HPLC du peptide Sc

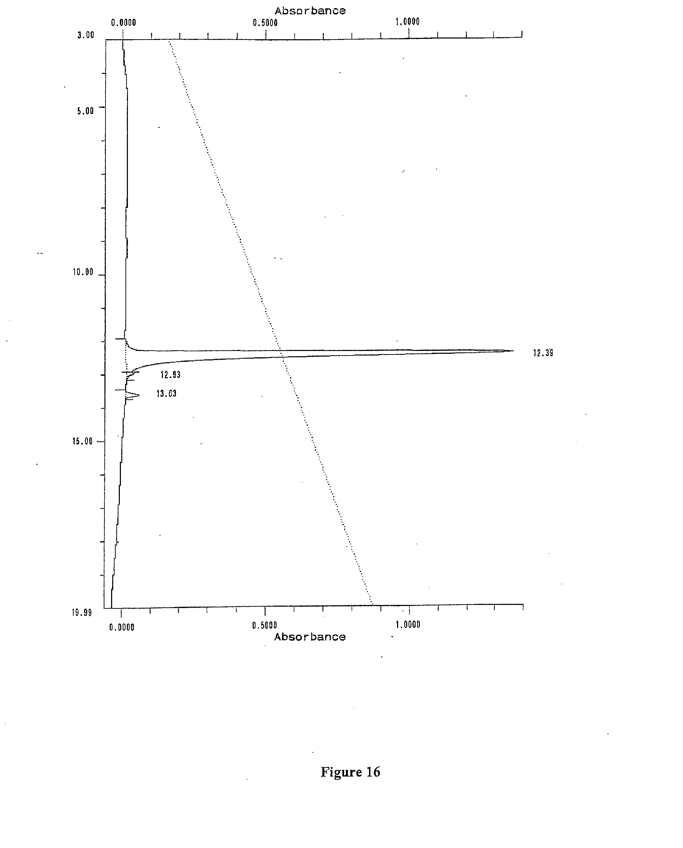

- Figure 16 : profil HPLC du peptide ScP

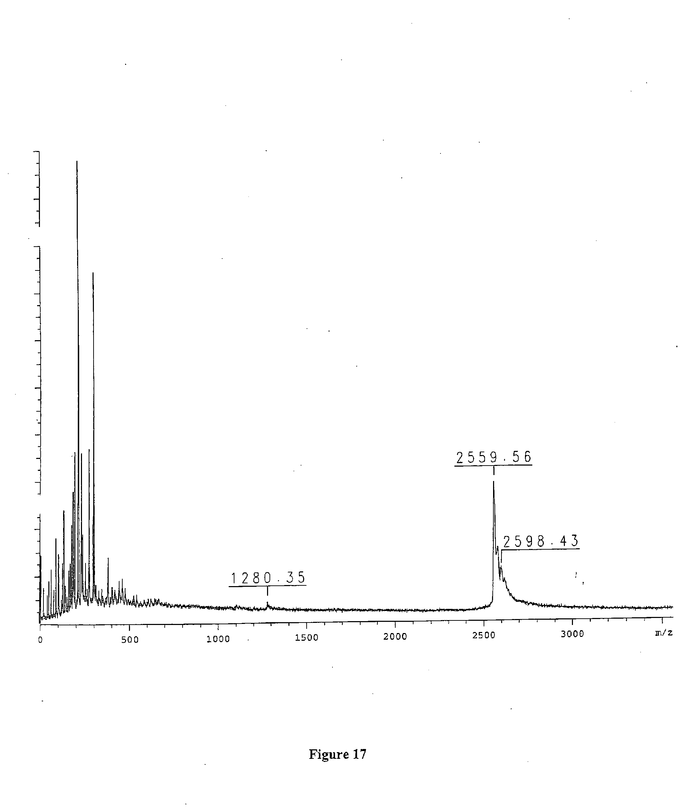

- Figure 17 : spectre de masse du peptide Sc

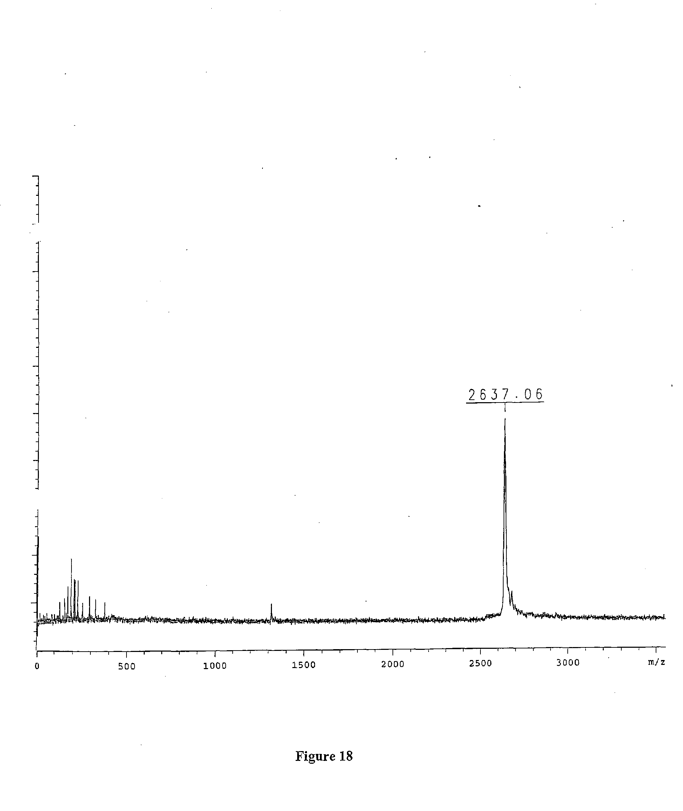

- Figure 18 : spectre de masse du peptide ScP
- Figure 19 : liaison des peptides 131-151 et P140 aux molécules du complexe majeur d'histocompatibilité de classe II humaines ; les résultats sont exprimés en pourcentage de liaison des peptides aux molécules HLA-DR1, -DR4 et DR11 ; le pourcentage d'inhibition est calculé en fonction des valeurs de DO mesurées en présence de différentes concentrations de peptides 131-151 et P140 (0.01-100µM).

### Modes de réalisation particuliers

1. Utilisation de peptides comprenant des épitopes de protéines du soi des mammifères reconnus par des anticorps produits par le système immunitaire des mammifères atteints de pathologies auto-immunes, et, le cas échéant, par les cellules T auxiliaires desdits mammifères, lesdits épitopes étant tels que l'un au moins de leurs acides aminés comporte une modification de type post-traductionnel, pour la préparation d'un médicament destiné à la prévention ou au traitement desdites pathologies auto-immunes.
2. Utilisation de peptides selon la revendication 1, comprenant des épitopes dont l'un au moins de leurs acides aminés est modifié de manière à ce qu'il soit sous une forme phosphorylée, ou acétylée.
3. Utilisation selon la revendication 1 ou 2, de peptides comprenant des épitopes issus des protéines d'origine humaine ou animale définies dans la revendication 1, choisies parmi les nucléoprotéines, les protéines du nucléosome, du splicéosome, de la particule ribonucléoprotéique Ro, ou du ribososme.
4. Utilisation de peptides selon l'une des revendications 1 à 3, pour la préparation d'un médicament destiné à la prévention ou au traitement :
   - de pathologies auto-immunes de la famille des connectivites (maladies systémiques non spécifiques d'organes), telles que le lupus érythémateux disséminé (LED), la polyarthrite rhumatoïde, la connectivite mixte, le syndrome de Sjögren, ou l'arthrite chronique juvénile,
   - ou de pathologies auto-immunes spécifiques d'organes, telles que la sclérose en plaques, le diabète insulino-dépendant, la maladie de Crohn, ou les maladies bulleuses.
5. Utilisation de peptides selon l'une des revendications 1 à 4, pour la préparation d'un médicament destiné à la prévention ou au traitement du LED.
6. Utilisation selon la revendication 5, de peptides comprenant des épitopes issus de la protéine humaine ou murine U1-70K du splicéosome.
7. Utilisation selon la revendication 5 ou 6, de peptides comprenant la séquence délimitée par les acides aminés 131 et 151 de la protéine U1-70K humaine ou murine, et correspondant à la séquence SEQ ID NO : 1 suivante :
   RIHMVYSKRSGKPRGYAFIEY
   dans laquelle l'un au moins des acides aminés comporte une modification de type post-traductionnelle, notamment par phosphorylation, ou acétylation.
8. Utilisation selon l'une des revendications 5 à 7, de peptides comprenant la séquence SEQ ID NO : 1 dans laquelle l'un au moins des résidus sérine en position 7 ou 10 est phosphorylé, et/ou l'un au moins des résidus lysine en position 8 ou 12 est acétylé.
9. Utilisation selon l'une des revendications 5 à 8, de peptides comprenant la séquence SEQ ID NO : 1 dans laquelle :
   - la sérine en position 7 est phosphorylée,
   - et/ou la sérine en position 10 est phosphorylée,
   - et/ou la lysine en position 8 est acétylée,
   - et/ou la lysine en position 12 est acétylée.
10. Utilisation selon l'une des revendications 5 à 9, de peptides choisis parmi les suivants :
   - la séquence SEQ ID NO : 1 dans laquelle la sérine en position 7 est phosphorylée,
   - la séquence SEQ ID NO : 1 dans laquelle la sérine en position 7 ainsi que la sérine en position 10 sont phosphorylées,
   - la séquence SEQ ID NO : 1 dans laquelle la sérine en position 7 est phosphorylée, et la lysine en position 8 est acétylée,
   - la séquence SEQ ID NO : 1 dans laquelle la sérine en position 7 est phosphorylée, et la lysine en position 12 est acétylée,
   - la séquence SEQ ID NO : 1 dans laquelle la sérine en position 7 est phosphorylée, et la lysine en position 8 ainsi que la lysine en position 12 sont acétylées,
   - la séquence SEQ ID NO : 1 dans laquelle la sérine en position 7 ainsi que la sérine en position 10 sont phosphorylées, et la lysine en position 8 est acétylée,
   - la séquence SEQ ID NO : 1 dans laquelle la sérine en position 7 ainsi que la sérine en position 10 sont phosphorylées, et la lysine en position 12 est acétylée,
   - la séquence SEQ ID NO : 1 dans laquelle la sérine en position 7 ainsi que la sérine en position 10 sont phosphorylées, et la lysine en position 8 ainsi que la lysine en position 12 sont acétylées,
   - la séquence SEQ ID NO : 1 dans laquelle la sérine en position 10 est phosphorylée,
   - la séquence SEQ ID NO : 1 dans laquelle la sérine en position 10 est phosphorylée, et la lysine en position 8 est acétylée,
   - la séquence SEQ ID NO : 1 dans laquelle la sérine en position 10 est phosphorylée, et la lysine en position 12 est acétylée,
   - la séquence SEQ ID NO : 1 dans laquelle la sérine en position 10 est phosphorylée, et la lysine en position 8 ainsi que la lysine en position 12 sont acétylées,
   - la séquence SEQ ID NO : 1 dans laquelle la lysine en position 8 est acétylée,
   - la séquence SEQ ID NO : 1 dans laquelle la lysine en position 12 est acétylée,
   - la séquence SEQ ID NO : 1 dans laquelle la lysine en position 8 et la lysine en position 12 sont acétylées.
11. Utilisation selon l'une des revendications 5 à 10, de peptides choisis parmi les suivants :
   - la séquence SEQ ID NO : 1 dans laquelle la sérine en position 7 est phosphorylée,
   - la séquence SEQ ID NO : 1 dans laquelle la sérine en position 10 est phosphorylée,
   - la séquence SEQ ID NO : 1 dans laquelle la lysine en position 8 est acétylée,
   - la séquence SEQ ID NO : 1 dans laquelle la lysine en position 12 est acétylée,
   - la séquence SEQ ID NO : 1 dans laquelle la lysine en position 8 et la lysine en position 12 sont acétylées.
12. Composition pharmaceutique caractérisée en ce qu'elle comprend au moins un peptide choisi parmi ceux définis dans l'une des revendications 1 à 11, en association avec un véhicule pharmaceutiquement acceptable.
13. Composition pharmaceutique selon la revendication 12, caractérisée en ce qu'elle comprend au moins un peptide choisi parmi ceux comprenant la séquence SEQ ID NO : 1 dans laquelle l'un au moins des acides aminés comporte une modification de type post-traductionnel, notamment par phosphorylation, ou acétylation..
14. Composition pharmaceutique selon la revendication 12 ou 13, caractérisée en ce qu'elle comprend au moins un peptide choisi parmi ceux comprenant la séquence SEQ ID NO : 1 dans laquelle l'un au moins des résidus sérine en position 7 ou 10 est phosphorylé, et/ou l'un au moins des résidus lysine en position 8 ou 12 est acétylé.
15. Composition pharmaceutique selon l'une des revendications 12 à 14, caractérisée en ce qu'elle comprend au moins un peptide choisi parmi ceux comprenant la séquence SEQ ID NO : 1 dans laquelle :
   - la sérine en position 7 est phosphorylée,
   - et/ou la sérine en position 10 est phosphorylée,
   - et/ou la lysine en position 8 est acétylée,
   - et/ou la lysine en position 12 est acétylée.
16. Composition pharmaceutique selon l'une des revendications 13 à 15, caractérisée en ce qu'elle comprend au moins un peptide choisi parmi les suivants :
   - la séquence SEQ ID NO : 1 dans laquelle la sérine en position 7 est phosphorylée,
   - la séquence SEQ ID NO : 1 dans laquelle la sérine en position 7 ainsi que la sérine en position 10 sont phosphorylées,
   - la séquence SEQ ID NO : 1 dans laquelle la sérine en position 7 est phosphorylée, et la lysine en position 8 est acétylée,
   - la séquence SEQ ID NO : 1 dans laquelle la sérine en position 7 est phosphorylée, et la lysine en position 12 est acétylée,
   - la séquence SEQ ID NO : 1 dans laquelle la sérine en position 7 est phosphorylée, et la lysine en position 8 ainsi que la lysine en position 12 sont acétylées,
   - la séquence SEQ ID NO : 1 dans laquelle la sérine en position 7 ainsi que la sérine en position 10 sont phosphorylées, et la lysine en position 8 est acétylée,
   - la séquence SEQ ID NO : 1 dans laquelle la sérine en position 7 ainsi que la sérine en position 10 sont phosphorylées, et la lysine en position 12 est acétylée,
   - la séquence SEQ ID NO : 1 dans laquelle la sérine en position 7 ainsi que la sérine en position 10 sont phosphorylées, et la lysine en position 8 ainsi que la lysine en position 12 sont acétylées,
   - la séquence SEQ ID NO : 1 dans laquelle la sérine en position 10 est phosphorylée,
   - la séquence SEQ ID NO : 1 dans laquelle la sérine en position 10 est phosphorylée, et la lysine en position 8 est acétylée,
   - la séquence SEQ ID NO : 1 dans laquelle la sérine en position 10 est phosphorylée, et la lysine en position 12 est acétylée,
   - la séquence SEQ ID NO : 1 dans laquelle la sérine en position 10 est phosphorylée, et la lysine en position 8 ainsi que la lysine en position 12 sont acétylées,
   - la séquence SEQ ID NO : 1 dans laquelle la lysine en position 8 est acétylée,
   - la séquence SEQ ID NO : 1 dans laquelle la lysine en position 12 est acétylée,
   - la séquence SEQ ID NO : 1 dans laquelle la lysine en position 8 et la lysine en position 12 sont acétylées.
17. Composition pharmaceutique selon l'une des revendications 13 à 16, caractérisée en ce qu'elle comprend au moins un peptide choisi parmi les suivants :
   - la séquence SEQ ID NO : 1 dans laquelle la sérine en position 7 est phosphorylée,
   - la séquence SEQ ID NO : 1 dans laquelle la sérine en position 10 est phosphorylée,
   - la séquence SEQ ID NO : 1 dans laquelle la lysine en position 8 est acétylée,
   - la séquence SEQ ID NO : 1 dans laquelle la lysine en position 12 est acétylée,
   - la séquence SEQ ID NO : 1 dans laquelle la lysine en position 8 et la lysine en position 12 sont acétylées.
18. Composition pharmaceutique selon l'une des revendications 13 à 17, caractérisée en ce qu'elle se présente sous une forme administrable par voie systémique, ou non invasive.
19. Composition pharmaceutique selon l'une des revendications 13 à 18, caractérisée en ce que les doses journalières de peptides chez l'homme sont d'environ 100 ng à environ 5 mg.
20. Peptides comprenant la séquence délimitée par les acides aminés 131 et 151 de la protéine U1-70K humaine, ou murine, et correspondant à la séquence SEQ ID NO : 1 suivante :
   RIHMVYSKRSGKPRGYAFIEY
   dans laquelle l'un au moins des acides aminés est phosphorylé, ou acétylé.
21. Peptides selon la revendication 20, comprenant la séquence SEQ ID NO : 1 dans laquelle l'un au moins des résidus sérine en position 7 ou 10 est phosphorylé, et/ou l'un au moins des résidus lysine en position 8 ou 12 est acétylé.
22. Peptides selon la revendication 20 ou 21, comprenant la séquence SEQ ID NO : 1 dans laquelle :
   - la sérine en position 7 est phosphorylée,
   - et/ou la sérine en position 10 est phosphorylée,
   - et/ou la lysine en position 8 est acétylée,
   - et/ou la lysine en position 12 est acétylée.
23. Peptides selon l'une des revendications 20 à 22, choisis parmi les suivants :
   - la séquence SEQ ID NO : 1 dans laquelle la sérine en position 7 est phosphorylée,
   - la séquence SEQ ID NO : 1 dans laquelle la sérine en position 7 ainsi que la sérine en position 10 sont phosphorylées,
   - la séquence SEQ ID NO : 1 dans laquelle la sérine en position 7 est phosphorylée, et la lysine en position 8 est acétylée,
   - la séquence SEQ ID NO : 1 dans laquelle la sérine en position 7 est phosphorylée, et la lysine en position 12 est acétylée,
   - la séquence SEQ ID NO : 1 dans laquelle la sérine en position 7 est phosphorylée, et la lysine en position 8 ainsi que la lysine en position 12 sont acétylées,
   - la séquence SEQ ID NO : 1 dans laquelle la sérine en position 7 ainsi que la sérine en position 10 sont phosphorylées, et la lysine en position 8 est acétylée,
   - la séquence SEQ ID NO : 1 dans laquelle la sérine en position 7 ainsi que la sérine en position 10 sont phosphorylées, et la lysine en position 12 est acétylée,
   - la séquence SEQ ID NO : 1 dans laquelle la sérine en position 7 ainsi que la sérine en position 10 sont phosphorylées, et la lysine en position 8 ainsi que la lysine en position 12 sont acétylées,
   - la séquence SEQ ID NO : 1 dans laquelle la sérine en position 10 est phosphorylée,
   - la séquence SEQ ID NO : 1 dans laquelle la sérine en position 10 est phosphorylée, et la lysine en position 8 est acétylée,
   - la séquence SEQ ID NO : 1 dans laquelle la sérine en position 10 est phosphorylée, et la lysine en position 12 est acétylée,
   - la séquence SEQ ID NO : 1 dans laquelle la sérine en position 10 est phosphorylée, et la lysine en position 8 ainsi que la lysine en position 12 sont acétylées,
   - la séquence SEQ ID NO : 1 dans laquelle la lysine en position 8 est acétylée,
   - la séquence SEQ ID NO : 1 dans laquelle la lysine en position 12 est acétylée,
   - la séquence SEQ ID NO : 1 dans laquelle la lysine en position 8 et la lysine en position 12 sont acétylées.
24. Peptides selon l'une des revendications 20 à 23, choisis parmi les suivants :
   - la séquence SEQ ID NO : 1 dans laquelle la sérine en position 7 est phosphorylée,
   - la séquence SEQ ID NO : 1 dans laquelle la sérine en position 10 est phosphorylée,
   - la séquence SEQ ID NO : 1 dans laquelle la lysine en position 8 est acétylée,
   - la séquence SEQ ID NO : 1 dans laquelle la lysine en position 12 est acétylée,
   - la séquence SEQ ID NO : 1 dans laquelle la lysine en position 8 et la lysine en position 12 sont acétylées.

## Revendications

1. Peptides comprenant la séquence délimitée par les acides aminés 131 et 151 de la protéine U1-70K humaine, ou murine, et correspondant à la séquence SEQ ID NO : 1 suivante :
RIHMVYSKRSGKPRGYAFIEY
dans laquelle l'un au moins des acides aminés est phosphorylé, ou acétylé et les sels de ceux-ci.

2. Peptides ou les sels de ceux-ci selon la revendication 1, comprenant la séquence SEQ ID NO : 1 dans laquelle l'un au moins des résidus sérine en position 7 ou 10 est phosphorylé, et/ou l'un au moins des résidus lysine en position 8 ou 12 est acétylé.

3. Peptides ou les els de ceux-ci selon la revendication 1 ou 2, consistant en la séquence SEQ ID NO : 1 dans laquelle :
- la sérine en position 7 est phosphorylée,
- et/ou la sérine en position 10 est phosphorylée,
- et/ou la lysine en position 8 est acétylée,
- et/ou la lysine en position 12 est acétylée.

4. Peptides ou les sels de ceux-ci selon l'une des revendications 1 à 3, choisis parmi les suivants :
- la séquence SEQ ID NO : 1 dans laquelle la sérine en position 7 est phosphorylée,
- la séquence SEQ ID NO : 1 dans laquelle la sérine en position 7 ainsi que la sérine en position 10 sont phosphorylées,
- la séquence SEQ ID NO : 1 dans laquelle la sérine en position 7 est phosphorylée, et la lysine en position 8 est acétylée,
- la séquence SEQ ID NO : 1 dans laquelle la sérine en position 7 est phosphorylée, et la lysine en position 12 est acétylée,
- la séquence SEQ ID NO : 1 dans laquelle la sérine en position 7 est phosphorylée, et la lysine en position 8 ainsi que la lysine en position 12 sont acétylées,
- la séquence SEQ ID NO : 1 dans laquelle la sérine en position 7 ainsi que la sérine en position 10 sont phosphorylées, et la lysine en position 8 est acétylée,
- la séquence SEQ ID NO : 1 dans laquelle la sérine en position 7 ainsi que la sérine en position 10 sont phosphorylées, et la lysine en position 12 est acétylée,
- la séquence SEQ ID NO : 1 dans laquelle la sérine en position 7 ainsi que la sérine en position 10 sont phosphorylées, et la lysine en position 8 ainsi que la lysine en position 12 sont acétylées,
- la séquence SEQ ID NO : 1 dans laquelle la sérine en position 10 est phosphorylée,
- la séquence SEQ ID NO : 1 dans laquelle la sérine en position 10 est phosphorylée, et la lysine en position 8 est acétylée,
- la séquence SEQ ID NO : 1 dans laquelle la sérine en position 10 est phosphorylée, et la lysine en position 12 est acétylée,
- la séquence SEQ ID NO : 1 dans laquelle la sérine en position 10 est phosphorylée, et la lysine en position 8 ainsi que la lysine en position 12 sont acétylées,
- la séquence SEQ ID NO : 1 dans laquelle la lysine en position 8 est acétylée,
- la séquence SEQ ID NO : 1 dans laquelle la lysine en position 12 est acétylée,
- la séquence SEQ ID NO : 1 dans laquelle la lysine en position 8 et la lysine en position 12 sont acétylées.

5. Peptides ou les sels de ceux-ci selon l'une des revendications 1 à 4, choisis parmi les suivants :
- la séquence SEQ ID NO : 1 dans laquelle la sérine en position 7 est phosphorylée,
- la séquence SEQ ID NO : 1 dans laquelle la sérine en position 10 est phosphorylée,
- la séquence SEQ ID NO : 1 dans laquelle la lysine en position 8 est acétylée,
- la séquence SEQ ID NO : 1 dans laquelle la lysine en position 12 est acétylée,
- la séquence SEQ ID NO : 1 dans laquelle la lysine en position 8 et la lysine en position 12 sont acétylées.

6. Peptides ou les sels de ceux-ci selon la revendication 1 comprenant la séquence SEQ ID NO : 1 dans laquelle la sérine en position 10 est phosphorylée.

7. Peptide ou les sels de celui-ci selon la revendication 6 consistant en la séquence SEQ ID NO : 1 dans laquelle la sérine en position 10 est phosphorylée.

8. Composition pharmaceutique comprenant au moins un peptide ou un sel de celui-ci choisi parmi ceux définis dans l'une des revendications 1 à 7 en combinaison avec un véhicule pharmaceutiquement acceptable.

9. Composition pharmaceutique selon la revendication 8, **caractérisée en ce qu'**elle se présente sous une forme administrable par voie systémique, ou non invasive.

10. Utilisation de peptides ou des sels de ceux-ci tels que définis dans l'une des revendications 1 à 7, pour la préparation d'un médicament destiné à la prévention ou au traitement de pathologies auto-immunes.

11. Utilisation selon la revendication 10 dans laquelle les pathologies auto-immunes sont choisies parmi :
- les pathologies auto-immunes de la famille des connectivites (maladies systémiques non spécifiques d'organes), telles que le lupus érythémateux disséminé (LED), la polyarthrite rhumatoïde, la connectivite mixte, le syndrome de Sjögren, ou l'arthrite chronique juvénile, ou
- les pathologies auto-immunes spécifiques d'organes, telles que la sclérose en plaques, le diabète insulino-dépendant, la maladie de Crohn, ou les maladies bulleuses.

12. Utilisation selon la revendication 11 dans laquelle la pathologie auto-immune est la LED.

13. Utilisation selon l'une quelconque des revendications 10 à 12 dans laquelle la prévention ou le traitement comprend l'administration à des doses journalières chez l'homme de peptides ou des sels de ceux-ci d'environ 100 ng à environ 5 mg.

14. Peptides ou les sels de ceux-ci selon l'une quelconque des revendications 1 à 7, pour leur utilisation dans la prévention ou le traitement de pathologies auto-immunes.

15. Peptides ou les sels de ceux-ci selon la revendication 14 dans laquelle les pathologies auto-immunes sont choisies parmi :
- les pathologies auto-immunes de la famille des connectivites (maladies systémiques non spécifiques d'organes), telles que le lupus érythémateux disséminé (LED), la polyarthrite rhumatoïde, la connectivite mixte, le syndrome de Sjögren, ou l'arthrite chronique juvénile, ou
- les pathologies auto-immunes spécifiques d'organes, telles que la sclérose en plaques, le diabète insulino-dépendant, la maladie de Crohn, ou les maladies bulleuses.

16. Peptides ou les sels de ceux-ci selon la revendication 15 dans laquelle la pathologie auto-immune est le LED.

17. Peptides ou les sels de ceux-ci selon l'une des revendications 14 à 16, dans laquelle la prévention ou le traitement comprend l'administration à des doses journalières chez l'homme de peptides ou des sels de ceux-ci d'environ 100 ng à environ 5 mg.
